Europäisches Patentamt

European Patent Office

Office européen des brevets

(19)

(11) **EP 1 264 174 B1**

(12) **EUROPÄISCHE PATENTSCHRIFT**

(45) Veröffentlichungstag und Bekanntmachung des Hinweises auf die Patenterteilung:
**27.04.2005 Patentblatt 2005/17**

(51) Int Cl.$^7$: **G01N 33/00**, G01N 27/12

(21) Anmeldenummer: 01913760.3

(86) Internationale Anmeldenummer:
**PCT/EP2001/000689**

(22) Anmeldetag: **23.01.2001**

(87) Internationale Veröffentlichungsnummer:
**WO 2001/069234 (20.09.2001 Gazette 2001/38)**

(54) **ELEKTRONISCHE EINRICHTUNG ZUR LEITWERTERFASSUNG**

ELECTRONIC DEVICE FOR DETECTING CONDUCTANCE

DISPOSITIF ELECTRONIQUE POUR MESURER LA CONDUCTANCE

(84) Benannte Vertragsstaaten:
**AT BE CH CY DE DK ES FI FR GB GR IE IT LI LU MC NL PT SE TR**

(30) Priorität: **14.03.2000 DE 10012313**

(43) Veröffentlichungstag der Anmeldung:
**11.12.2002 Patentblatt 2002/50**

(73) Patentinhaber: **Forschungszentrum Karlsruhe GmbH**
**76133 Karlsruhe (DE)**

(72) Erfinder:
• **BLANK, Thomas**
**76187 Karlsruhe (DE)**

• **DEIMLING, Berthold**
**76646 Bruchsal (DE)**
• **FROMHEIN, Olaf**
**76646 Bruchsal (DE)**
• **WIEDEWILT, Wolfgang**
**68789 St. Leon-Rot (DE)**

(56) Entgegenhaltungen:
**EP-A- 0 293 255       US-A- 5 783 154**
**US-A- 5 945 069**

• **PATENT ABSTRACTS OF JAPAN vol. 009, no. 156 (P-368), 29. Juni 1985 (1985-06-29) -& JP 60 031049 A (MATSUSHITA DENKO KK), 16. Februar 1985 (1985-02-16)**

## Beschreibung

Die Erfindung betrifft eine elektronische Einrichtung zur Leitwerterfassung von stromdurchflossenen Bauelementen. Sind diese Bauelemente hauptsächlich halbleitende Sensoren, Gassensoren z.B., die ihren Leitwert in einer Atmosphäre unterschiedlicher Gaskomponenten gasspezifisch ändern, dann lässt sich damit eine Atmosphäre auf ihre Gaskomponenten hin untersuchen, wenn ein an eine Signalverarbeitungseinrichtung angeschlossenes Array solcher Gassensoren darin exponiert ist.

In der Nahrungs- und Genussmittelindustrie, in Fährzeugen, Küchen, Häusern und im Umweltschutzbereich wird Luft auf verschiedene, gasförmige Stoffe hin überwacht. Als Messeinrichtung zur Ermittlung gasförmiger Bestandteile in der Luft wird ein an eine Auswerteelektronik angeschlossener Sensorchip verwendet. Ein solcher besteht aus einer Anzahl solcher Gassensoren,die auf halbleitenden Metalloxiden aufgebracht sind, deren elektrischer Widerstand von der Zusammensetzung der Luft abhängt.

Zwei Effekte werden ausgenutzt:

- Die spezifische Reaktion von Gasgruppen mit einem Metall. Wolframoxid ist beispielsweise gut für die Detektion von $CO_2$ Zinndioxid gut für Stickoxide.
- Die Reaktion hängt von der Temperatur des Sensorelementes ab. Die einzelnen Sensorelemente werden bei unterschiedlichen Temperaturen betrieben. Jede Gaskomponente reagiert bei unterschiedlichen Temperaturen mit anderen Leitwertänderungen, so dass aus dem Widerstandsprofil über der Temperatur auf die Substanz geschlossen werden-kann.

Ein derartiger Sensortyp ist aus Zinnoxid als halbleitendem Material aufgebaut und in der DE 44 23 289 beschrieben. Der Widerstandsbereich, der sich aufgrund unterschiedlicher Gaskomponenten einstellen kann, erstreckt sich von Werten im $M\Omega$-Bereich, etwa 300 $M\Omega$, bis runter in den $k\Omega$-Bereich, ca. 1 $k\Omega$.

Zur Ermittlung des aktuellen Widerstands eines Sensors wird heute meist aus dem Sensor-Array in der zu untersuchenden Atmosphäre ein Referenzstrom $I_{mess}$ durch diesen einzigen, über einen Multiplexer an diese Quelle gelegten Sensor geleitet, der an ihm einen Spannungsabfall hervorruft. Dieser wird an den Eingang eines Verstärkers gelegt, an dessen Ausgang eine entsprechende Spannung $U_{mess}$ ansteht. Der aktuelle Widerstand des gerade aktivierten Sensors ist

$$R_m = 1/V * U_{mess}/I_{mess}$$

V ist der Verstärkungsfaktor des Verstärkers. Der Widerstandswert des jeweiligen Sensors auf dem Sensorchip wird sequentiell ermittelt, in dem mit dem Multiplexer der Referenzstrom durch den jeweiligen Sensor geführt wird.

Bei dieser Art Widerstandsermittlung besteht folgende Problematik:

Auf der Sensoroberfläche befinden sich eine Vielzahl Sensoren je nach Bauart bis zu 40 davon. Die hohe notwendige Messbereichsdynamik zur Erfassung der Widerstandswerte von 1 $k\Omega$ bis 300 $M\Omega$ wird durch Umschalten der Referenzströme und der Variation der Verstärkung des Instrumentenverstärkers erreicht. Schaltungskapazitäten in Verbindung mit hohen Messwiderständen führen zu Einschwingzeiten bis zu 10 msec. Die Schalterwiderstände des Multiplexers haben Widerstände im $G\Omega$-Bereich. Durch die Parallelschaltung vieler solcher Widerstände zu dem jeweiligen Messwiderstand entsteht eine Verfälschung des tatsächlichen Wertes.

Um 40 Sensoren eines Sensor-Arrays auf einem Chip auszumessen, sind ca. 1,6 sec nötig. Diese Zeit erhöht sich mit der Anzahl Messbereiche, die verwendet werden, und jedes Mal muss die Spannung aufgrund der Schalt- und parasitären Kapazitäten einschwingen. Bei zwei unterschiedlichen Verstärkungsfaktoren in der dem Sensorchip nachgeschalteten Elektronik und vier verschiedenen Referenzströmen, die zur Abdeckung des Messwiderstandsbereichs von 1 $k\Omega$ bis 300 $M\Omega$ verwendet werden müssen, werden für einen umfassenden Messvorgang 12,8 sec benötigt. Das ist bei der geforderten Anzeige eines Gefahrenzustandes zu lang, das auch mit Rechnerverarbeitung nicht verkürzt werden kann.

In der EPA 0 293 255 wird ein Instrument zur Detektion von Zielgasen beschrieben. Der Sensor ist temperaturstabilisiert. Die Gassensoren in der Sensoranordnung haben eine erhöhte Packungsdichte durch kleinere Sensoren und eine längere Lebensdauer durch Reduktion der Leistungsaufnahme. Ein programmierbarer Kreis wirkt als programmierbarer Widerstand-Temperatur-Regler in einer Wheatstone schen Brückenschaltung. Ein verstärkender AD-Wandlerkreis steuert das Verhältnis von Spannung und Strom, das als ein Widerstand-Äquivalent arbeitet. Der programmierbare Kreis ist an ein Rechner angeschlossen, um eine selektive Temperatursteuerung für etwa 250 Einstellungen bereitstellen zu können.

Der Erfindung liegt die Aufgabe zugrunde, die Detektion von unterschiedlichen Gaskomponenten in einer Atmosphäre zeitlich kürzer wie bisher und für das notwendig rasche Anzeigen von Gefahrzustanden sicher zu detektieren.

Die Aufgabe wird im Grundsatz durch eine elektronische Einrichtung zur Leitwerterfassung stromdurchflossener Bauelemente unterschiedlicher Leitwerte gemäß Anspruch 1 gelöst: 2*2n bestrombare Bauelemente sind hierzu auf einem Träger zu einer exponierbaren Anordnung angebracht und elektrisch zu einem Ring geschaltet. n ist eine natürliche Zahl derart, dass die notwendige Anzahl 2*2n solcher Bauelemente sicher besteht.

An diese Ringstruktur koppelt eine Referenzspannungsquelle $V_{ref}$ über 2k Umschalter $U_k$ an und treibt einen leitwertabhängigen Strom durch das jeweils angeschlossene Bauelement. Diese Ströme werden jeweils einem elektronischen Kanal $V_1$ zugeführt, in dem ein Strom-Spannungswandlung vorgenommen wird. Hierzu hat die Einrichtung 21 elektronische Kanäle $V_1$, mit denen gleichzeitig 21 Bauelemente auf ihren Leitwert hin überprüfbar sind. Die jeweils zwei Ausgänge der 21 Kanäle werden auf die Eingangsleiste eines AD-Wandlers, zweckmäßigerweise auf die Eingangsleiste des AD-Wandlers eines Mikrocontrollers, $\mu$C, gelegt, in dem die jeweiligen Ausgangssignale erfasst, weiterprozessiert und gegebenenfalls für Signalzwecke weiter verwendet werden.

k und 1 sind ebenfalls natürliche Zahlen und stehen mit n in folgendem Zusammenhang:

$$2k*2l = 2*2n.$$

Somit ist eine Beziehung zwischen der Anzahl Bauelemente, der notwendigen Anzahl an Umschaltern $U_k$ und elektronischen Kanälen $V_1$ hergestellt. Eine darüber hinausgehende Anzahl an Umschaltern und Kanälen ist entsprechend redundant. Es gibt daher stets eine Anordnung von 4n Bauelementen, also einem ganzzahligen Vielfachen von vier.

Schaltungstechnisch ist der Ring aus 2*2n Bauelementen $R_m$ und $R_{Re}$ als ein Ring von Bauelementepaaren $R_{P2n}$ zu betrachten. In jedem Bauelementepaar $R_{P1}$ gibt es damit einen gemeinsamen Punkt der beiden zum Paar zusammengefassten Bauelemente. Dieser gemeinsame Punkt wird auf den Ausgang eines der verwendeten Umschalter so gelegt, dass ein Umschalter mit seinem Ausgang niemals an zwei unmittelbar benachbarten Bauelementepaare ankoppelt.

Aufgrund des oben aufgeführten algebraischen Zusammenhangs, ist die Anzahl Umschalter geradzahlig, mindestens gleich 2 und höchstens gleich der Anzahl Bauelementepaare; dazu komplementär die Anzahl Verstärkerketten. Wesentlich während des Betriebs der Einrichtung ist, dass in einem Messintervall nur einer von den 2k Umschaltern die Referenzspannung $V_{ref}$ an seinem Ausgang anliegen hat, alle andern haben ein Bezugspotential 0, das komplementäre Potential, anliegen, mit dem kein Strom durch die Bauelemente getrieben werden kann.

Der durch das jeweilige Bauelement getriebene Strom darf nur von einem elektronischen Kanal erfasst werden. Daher wird über logische Verknüpfung der gemeinsame Punkt zweier benachbarter Bauelementepaare entsprechend auf den negativen Eingang eines der Kanäle gelegt. Einfach ist die Verschaltung der Umschalter und Kanäle, wenn die hochstmögliche Anzahl Umschalter, nämlich 2n, und damit die kleinstmögliche Anzahl Kanäle, nämlich 2, oder umgekehrt, angeschlossen werden soll. Diese beiden Verschaltungsmöglichkeiten sind dann sogar die einzigen, wenn eine n Primzahl ist.

Soll die Einrichtung schnell sein, wird die Kanal- oder Verstärkerkettenanzahl maximal also gleich 2n sein und die Umschalteranzahl minimal, also zwei. Ist die Geschwindigkeit der Messwerterfassung nicht maßgebend, könnte es wirtschaftlicher sein, die höchstmögliche Anzahl Umschalter also 2n und die kleinste Anzahl Verstärkerketten also 2 zu verschalten.

Ist n keine Primzahl, dann gibt es außer den Faktorenpaaren

$$(k, l) = (1, n) \text{ und } (n, 1)$$

noch die möglichen Faktorenpaarbildungen (k, 1) und (1, k) dazwischen. Das beginnt mit n = 4, wo neben der Faktorenpaarbildung (1, 4) und (4, 1) noch das Faktorenpaar

$$(k, l) = (k, l) = (2, 2)$$

möglich ist. Für n = 6 gibt es erstmals zwei dazwischen liegende Faktorenpaare, nämlich

$$(k, l) = (2, 3) \text{ und } (3, 2).$$

Aus dieser Betrachtung geht auch die Belegungshäufigkeit des jeweiligen Ausgangs der Umschalter als auch die des jeweiligen Eingangs der Kanäle hervor. Ist k = l und l = n, dann ist die Belegungshäufigkeit des Ausgangs jeden Umschalters

$$2n/2k = n/k = n$$

und die des Eingangs jeden Kanals

$$2n/2l = n/l = 1.$$

Ist n nicht prim, so dass es dazwischen liegende Faktorenpaare (k, l) gibt, dann ist die Belegungshäufigkeit am Ausgang eines jeden Umschalters 1 und die am Eingang eines jeden Kanals komplementär, also k.

Um dafür die Verschaltung der 21 Kanäle zu finden, ist es zweckmäßig zu den 21 Kanälen noch 2(n-1) weitere fiktive Kanäle $V_{a, b, c, \ldots}$ einzuführen, so dass fiktiv eine Einfachbelegung der Kanaleingänge besteht. Über logische Verknüpfung wird dann festgelegt, welcher der 2(n-1) Kanäle mit den vorhandenen 21 Kanälen verbunden wird, woraus dann die Verschaltung des Rings mit den 21 Kanälen hervorgeht. Die Belegungshäufigkeit jeden Kanaleingangs ist, wie erläutert, k. Die Verschaltung ist bis auf die beiden Faktorenpaare (1, n) und (n, 1) sowie für n als Primzahl im allgemeinen nicht eindeutig. Daher werden die Ausgänge der Verstärker im AD-Wandler adressiert.

Die bisherigen Betrachtungen gehen davon aus, dass die Umschalter mit ihrem jeweiligen Ausgang zunächst reihum an die Bauelementepaare angeschlossen werden und dann über logische Verknüpfungen die Kanäle mit ihrem jeweiligen Messeingang reihum an die Verbindungen zwischen den Bauelementepaaren. Etwa vom Bestromungsvorgang her ist die Erstverschaltung der Umschalter plausibel. Die Vorgehensweise ist aber umgekehrt, also Erstverschaltung der Kanal- bzw. Verstärkereingänge, genauso möglich, und die Verschaltungs- und Anschlussüberlegungen gelten auch in dieser Richtung.

Für die Gassensorik bestehen die Bauelemente zumindest teilweise aus halbleitenden Sensoren, die ihren Leitwert in einer Atmosphäre unterschiedlicher Gaskomponenten gasspezifisch ändern. Der Rest der Bauelemente sind ohmsche Widerstände, sog. Referenzwiderstände, mit denen im Ring die Messwiderstände bzw. Gassensoren stets überprüft werden können und so Offsetspannungen und Fehlerströme im Sensoraufbau und in der Elektronik mit Hilfe des Mikrokontrollers kompensierbar werden. Sie haben daher einen festen, im allgemeinen jeweils einen unterschiedlichen ohmschen Widerstand, der in seinem Leitwert zumindest durch die Einflüsse der Gaskomponenten der ihn umgebenden Atmosphäre nicht beeinflusst wird (Anspruch 2).

Einfach und damit kostengünstig ist der Aufbau der Verstärkerkette mit ihrer unechten Differenzverstärkung der Auswertestufe zur Strom-Spannungswandlung. Die Verstärkerkette oder der elektronische Kanal besteht aus mindestens einem Verstärker, dessen Ausgang über einen Widerstand auf den negativen Eingang rückkcpppelt. Sie eignet sich für den Betrieb mit nur einer, hier positiven Versorgungsspannung. Die Ausgangsspannung der Verstärker in der Verstärkerkette ist dahin gehend begrenzt, als dadurch keine Schutzstrukturen für den nachgeschalteten AD-Wandler notwendig sind.

Die Einrichtung verwendet in wirtschaftlicher Weise, d.h. Umschalter und Kanäle zur Strom-Spannungswandlung in minimal notwendiger Anzahl bei entsprechender Priorität für die Zeit der Signalverarbeitung.

Sollte sich aus Anfangsüberlegungen heraus für n zunächst eine Primzahl n ≥ 5 als notwendig ergeben und die beiden einzigen Verschaltungen aufgrund der nur möglichen beiden Produktpaare (l, n) und (n, l) nicht erwünscht sein, so kann unter Akzeptanz von größer werdender Redundanz, Erhöhung der Bauelementeanzahl in 4.er Schritten, eine Zahl n zwischen dieser Primzahl und der folgenden Primzahl für die Schaltungsbildung herangezogen werden, da für diese dazwischenliegenden Zahlen ja mindestens ein Faktorenpaar (k, l) ≠ (l, n) und (n, l) existiert.

Die Erfindung wird im folgenden anhand der Zeichnung näher erläutert. Die Zeichnung besteht aus drei Figuren. Es zeigt:

Figur 1 die Verschaltung für das Produktpaar (1, 6),
Figur 2 eine Verschaltung für das Produktpaar (2, 3),
Figur 3 die Basisschaltung zur Erfassung eines Leitwerts.

Das Betrachtungsbeispiel besteht aus 24 Bauelementen $R_m$, $R_{Re}$. Damit ist n = 6. Wegen k*l = n, gibt es mindestens vier Verschaltungsmöglichkeiten der Umschalter und Verstärkerketten mit dem Ring, nämlich:

i) 1*6 ⇒ 2 Umschalter und 12 Verstärkerketten oder
ii) 2*3 ⇒ 4 Umschalter und 6 Verstärkerketten oder

iii) 3*2 ⇒ 6 Umschalter und 4 Verstärkerketten oder

iv) 6*1 ⇒ 12 Umschalter und 2 Verstärkerketten.

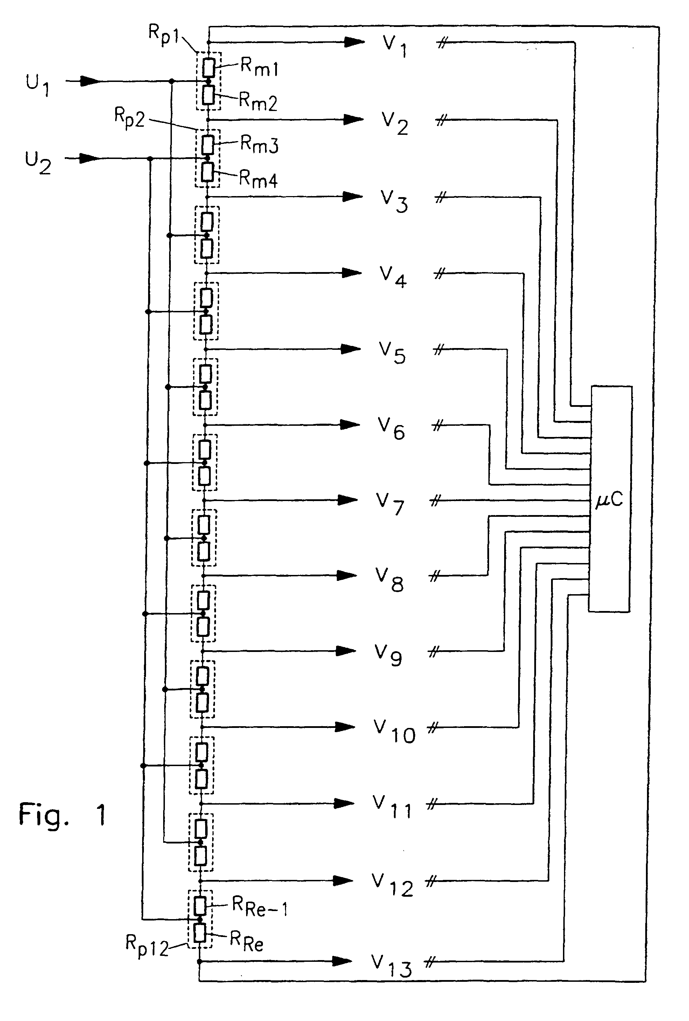

Schaltungstechnisch sind die beiden Verschaltungen i) (1, 6) und iv) (6, 1) eindeutig. Figur 1 zeigt die Verschaltung der beiden Umschalter $U_1$ und $U_2$ mit den 12 Kanälen $V_1$ bis $V_{12}$. Die beiden Umschalter sind reihum mit ihrem jeweiligen Ausgang an den Ring der aus den 24 Bauelementen $R_m$, $R_0$ gebildeten Bauelementepaare $R_{P1}$ bis $R_{P12}$ angeschlossen. Die 12 Kanäle $V_1$ bis $V_{12}$ sind mit ihrem jeweiligen Eingang auf die 12 Verbindungen zwischen den Bauelementepaaren gelegt. Damit ist jeder Kanaleingang einfach belegt und jeder Umschalterausgang 6-fach belegt.

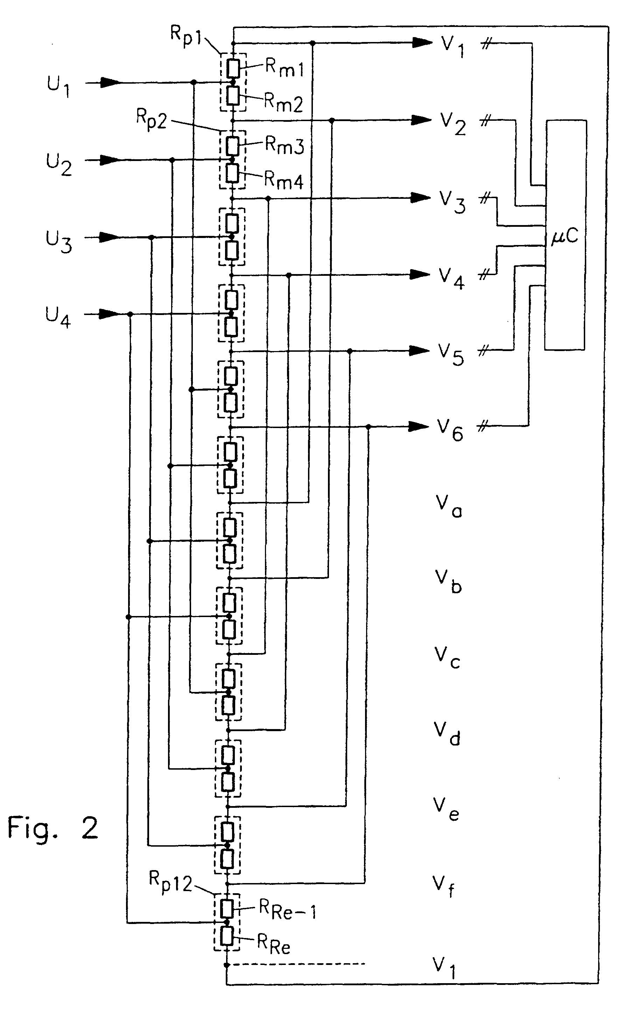

Für das Faktorenpaar (2, 3) zeigt Figur 2 eine von vier Verschaltungsmöglichkeiten. Der jeweilige Ausgang der 4 Umschalter $U_{1\,bis\,4}$ ist zunächst reihum an die Bauelementepaare gelegt und wird dadurch 6/2 = 3-mal belegt. Dann werden die vier Möglichkeiten über die 2 (6-3) = 6 fiktiven Kanäle $V_{a\,bis\,f}$ ermittelt. Die 6 notwendigen und die 6 fiktiven Kanäle verteilen sich reihum, mit ihrem jeweiligen Eingang einfach belegt, auf die 12 von den Bauelementepaaren gebildeten Verbindungen. Durch die Verknüpfung von Umschalterausgang - Bauelemtepaar - Kanal ergeben sich die Verbindungen der fiktiven mit den notwendigen Kanälen. Dabei ist in diesem Ausführungsbeispiel der fiktive Kanal $V_c$ stets an den notwendigen Kanal $V_3$ und der fiktive Kanal d stets an den notwendigen Kanal $V_4$ gekoppelt. Anders die fiktiven Kanäle $V_{a, b, e\,und\,f}$: bei ihnen gibt es jeweils zwei Möglichkeiten mit den notwendigen Kanälen $V_{1, 2, 5\,und\,6}$, die aber nicht willkürlich sind, vielmehr teilweise gegenseitige Ausschließungen fordern. In der folgenden Tabelle sind alle vier Verschaltungsmöglichkeiten I bis IV für das Faktorenpaar (2, 3) zusammengefasst.

| Tabelle der Verschaltungen für das Faktorenpaar (2, 3) | | | | |
|---|---|---|---|---|
| | I | II | III | IV |
| $V_a$ | $V_1$ | $V_1$ | $V_5$ | $V_5$ |
| $V_b$ | $V_2$ | $V_6$ | $V_2$ | $V_6$ |
| $V_c$ | $V_3$ | $V_3$ | $V_3$ | $V_3$ |
| $V_d$ | $V_4$ | $V_4$ | $V_4$ | $V_4$ |
| $V_e$ | $V_5$ | $V_5$ | $V_1$ | $V_1$ |
| $V_f$ | $V_6$ | $V_2$ | $V_6$ | $V_2$ |

Figur 2 zeigt die Verschaltungsmöglichkeit I. Die Umschalterausgangsbelegung ist 6/2 = 3-fach, die Kanaleingangsbelegung ist 6/3 = 2-fach.

Welche Aufbaumöglichkeit bei einer notwendigen Anzahl an Bauelementen schließlich ausgewählt wird, orientiert sich an Forderungen, die für diese grundsätzliche Betrachtungsweise nicht zwingend sind.

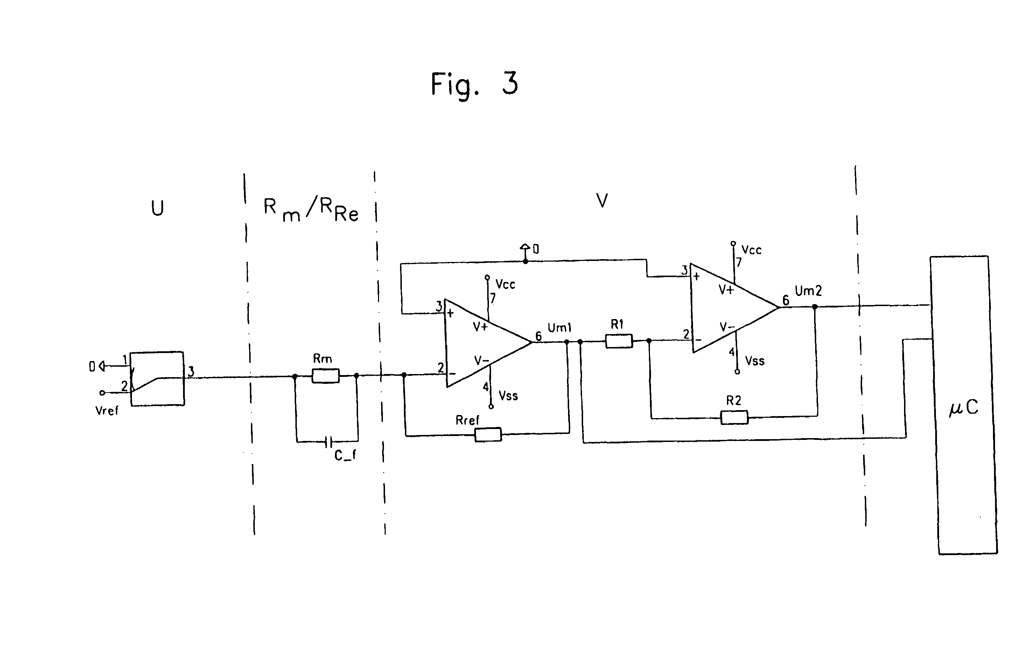

Fur die Messbereicherweiterung ist in Fig. 3 der Eingangsverstarkerstufe, der Strom-Spannungs-Wandlung, noch eine Differenzverstärkerstufe nachgeschaltet, deren positiver Eingang auf dem Bezugspotential 0 liegt und an deren negativen Eingang der Widerstand $R_1$ angeschlossen ist. Der Ausgang dieser Stufe $U_{m3}$, der einerseits einer der beiden Ausgänge des Kanals ist, koppelt über den Widerstand $R_2$ auf den negativen Eingang zurück. Bei Bedarf könnte noch eine weitere Differenzverstärkerstufe nachgeschaltet werden.

Mit dieser Schaltung für in der Fig. 3 nur ein Bauelement $R_{m1}$, dem halbleitenden, chemischen Gassensor, wird die Einschwingzeit bis zum stabilen Wert maßgeblich verkürzt. Die Schaltkapazitäten, die durch die zum Messwiderstand $R_{m1}$ parallel liegende Kapazität $C_f$ repräsentativ angedeutet ist, wird über den Innenwiderstand der einstellbaren Referenzspannungsquelle $V_{ref}$ durch den Umschalter U ge- und entladen. Der Messwiderstand $R_{m1}$ wird über den Umschalter U an die konstante Spannung $V_{ref}$ gelegt. Beim Einschalten wirkt die repräsentative Kapazität $C_f$ zunächst wie ein Kurzschluss. Sie wird wegen des hohen Stromes sehr schnell geladen, wodurch der Einschwingvorgang rasch abgeschlossen ist. Am Ausgang des Kanals liegen nahezu sofort die dem Messwiderstand oder Leitwert des Gassensors $R_{m1}$ entsprechenden Spannungen $U_{m1}$ und $U_{m3}$ zur Auswertung an. Für den Gassensor werden damit in Abhängigkeit des eingesetzten Verstärkertyps Einschwingzeiten im μsecbis msec-Bereich erreicht. Bei der Umschaltung vom Bezugspotential, das hier 0 V ist, auf die Spannung $V_{ref}$ läuft die entsprechende Aufladung ab.

Die Schaltung gemäß Figuren 1,2 und 3 kommt mit der unipolaren Versorgungsspannung von hier +5 V aus, wobei das Bezugspotential von 0 V auf einen beliebigen Wert zwischen 0 und +5 V gelegt werden muss. Negative Spannungen werden nicht gebraucht.

Mit den Umschaltern werden beim Messen die jeweils auszuwertenden Messwiderstände wahlweise an die Referenzspannung $V_{ref}$ oder das Bezugspotential 0 V gelegt. Durch die Stellung eines der vier Umschalter zum Anlegen der vorgegebenen Referenzspannung $V_{ref}$ und der komplementären Stellung, 0 V, der übrigen samt den Umschaltern zu den Konstantwiderständen wird der aktuelle Leitwert des entsprechenden Gassensors $R_{mi}$ durch die die Spannungen $U_{m1}$ und $U_{m3}$ an den beiden Ausgängen der Verstärkerkette dargestellt. Diese Werte stehen wegen der Komplementärstellung der übrigen Umschalter fast sofort an.

Die beiden Ausgangsspannungen $U_{m1}$ und $U_{m3}$ werden an den Eingang des AD-Wandler im Mikrocontroller $^{\mu}$C geführt. Mit dem Mikrocontroller wird der Messwiderstand bzw. dessen aktueller Leitwert unmittelbar rechnerisch ermittelt.

Da das System einfach und wirtschaftlich aufgebaut sein soll, aber dabei dennoch zuverlässig arbeiten soll, ist zur Erfassung irgend welcher Driften in der Funktion ständig ein Überprüfen des Systems notwendig. Die gasunempfindlichen Bauelemente $R_{Re}$ sind Bestandteil des Rings; sie lassen eine regelmäßige Überprüfung der einzelnen Gassensoren zu und ermöglichen Offsetspannungen in der Elektronik, insbesondere in den beiden Verstärkerketten, als auch Leckströme auf systemnotwendigen Platinen mit Hilfe des Mikrocontrollers $^{\mu}$C zu kompensieren.

Hierzu liegt ein ausgewählter Gassensor $R_m$ und einer der konstanten Widerstände $R_{Re}$ an der Spannung $V_{ref}$, die Ausgänge der übrigen Umschalter haben das komplementäre Potential 0. Statt des einen Gassensors $R_m$ und des Referenzwiderstandes $R_{Re}$ können das auch zwei Referenzwiderstände sein, die jeweils an einer einzelnen Leitwerterfassung liegen. Aus den Messwerten und den bekannten Widerstandswerten wird für jede Verstärkerkette $V_1$ der Fehler, Offset und Leckstrom ermittelt und daraufhin dann für die Gassensoren kompensiert. Jetzt lässt sich im Rechner der wahre Messwert $R_{mist}$ mit dem entsprechenden Konstantwiderstand $R_{Re}$ vermessen und damit mit Rechnerhilfe die Fehler der elektronischen Bausteine und der Leiterplatine leicht und ohne Hardware-Aufwand bekleidet zu der Messung kompensieren.

Es ist nützlich, die Versorgungsspannung der Verstärker in den beiden Verstärkerketten so zu wählen, dass der AD-Wandler des Mikrocontrollers $^{\mu}$C ohne zusätzliche elektronische Schutzstrukturen auskommt.

Ein weiterer Vorteil ist die Vermeidung von Leckströmen, die durch die Leckwiderstände der auf dem Bezugspotential liegenden Schalter entstehen. Dies, weil die Potentialdifferenz über dem Schalter verschwindet und dadurch kein Strom getrieben werden kann.

**Patentansprüche**

1.  Elektronische Einrichtung zur Leitwerterfassung, bestehend aus:

    2*2n elektrisch zu einem Ring geschalteten Bauelementen ($R_m$, $R_{Re}$), n ist eine beliebige natürliche Zahl, die im allgemeinen jeweils unterschiedlich elektrischen Leitwert haben,

    2k Umschaltern ($U_k$), k ist eine natürliche Zahl, die je nach Schalterstellung eines von zwei Potentialen, einer Referenzspannung ($V_{ref}$) und einem Bezugspotential (0), am Eingang an ihren Ausgang durchschalten,

    21, l ist eine natürliche Zahl, elektronischen Kanälen ($V_1$) zur Strom-Spannungs-Wandlung des durch ein angeschlossenes und gerade bestromtes Bauelement ($R_m$ oder $R_{Re}$) fließenden Stroms, wobei jeder Kanal ($V_1$) einen negativen und einen positiven Eingang sowie zwei Ausgänge hat,

    einem AD-Wandler ($^{\mu}$C), an dessen Eingangsleiste die jeweils zwei Ausgänge der 21 Kanäle gelegt sind, wobei:
    gilt:

    $$2k*2l = 2*2n,$$

    der Ausgang eines ersten Umschalters ($U_1$) am gemeinsamen Punkt zweier im Ring benachbarter Bauelemente ($R_{m1}$, $R_{m2}$), einem ersten Bauelementepaar ($R_{P1}$), liegt, der Ausgang des nächsten Umschalters ($U_2$) am gemeinsamen Punkt der nächsten zwei Bauelemente ($R_{m3}$, $R_{m4}$), des folgenden, zweiten Bauelementepaares ($R_{P2}$) im Ring usw. liegt, bis alle gemeinsamen Punkte der 2n Bauelementepaare ($R_{Pn}$) des Ringes sukzessive angeschlossen sind und damit der jeweilige Ausgang der 2k Umschalter ($U_k$) n/k-fach belegt ist,

    jeder elektronische Kanal ($V_1$) mit seinem negativen Eingang an einem gemeinsamen Punkt zweier benachbarter Bauelementepaare ($R_{P1}$, $R_{P1+1}$) angeschlossen ist, sein positiver Eingang stets auf dem Bezugspotential (0) liegt, und jeweils beide Ausgänge auf die Eingangsleiste eines AD-Wandlers ($^{\mu}$C) gelegt sind,

    die Referenzspannung ($V_{ref}$) während eines Messintervalls immer nur an einem Ausgang der 2k Umschalter

($U_k$) anliegt, so dass die daran angeschlossenen Bauelementepaare alleine davon bestromt werden, die übrigen Ausgänge in dieser Zeit auf dem Bezugspotential (0) liegen und der jeweils negative Eingang der 21 elektronischen Kanäle ($V_1$) so an den gemeinsamen Punkt zweier benachbarter Bauelementepaare ($R_{Pi}$, $R_{Pi+1}$) gelegt ist, dass auch bei mehrfacher Belegung des negativen Eingangs stets nur der Strom durch ein Bauelement ($R_m$ oder $R_{Re}$) pro elektronischen Kanal ($V_1$) gemessen wird,

n/k Bauelementepaare ($R_P$) gleichzeitig bestromt und damit 21 Bauelemente ($R_m$ und/oder $R_{Re}$)gleichzeitig gemessen werden.

**2.** Elektronische Einrichtung nach Anspruch 1,
**dadurch gekennzeichnet, dass**
die Bauelemente ($R_m$) halbleitende Sensoren sind, die ihren Leitwert in einer Atmosphäre unterschiedlicher Gaskomponenten gasspezifisch ändern, und
die Bauelemente ($R_{Re}$) ohmsche Widerstände, Referenzwiderstände, sind, die einen festen, im allgemeinen jeweils einen unterschiedlich ohmschen Widerstand haben, der in seinem Leitwert von einer umgebenden Atmosphäre nicht beeinflusst wird.

**3.** Elektronisches Einrichtung nach Anspruch 2,
**dadurch gekennzeichnet, dass**
in jeder Einrichtung der Eingang des jeweiligen Kanals ($V_1$) zuerst auf den negativen Eingang einer Verstärkerstufe zur Strom-Spannungswandlung führt, deren unmittelbarer Ausgang zum einen über einen ohmschen Widerstand ($R_{ref}$) auf ihren negativen Eingang rückkoppelt, zum andern einer der beiden Ausgänge des Kanals bildet und weiter über einen ohmschen Längswiderstand ($R_1$) bei ausreichendem Messbereich direkt den zweiten Ausgang oder noch eine Verstärkerkette aus mindestens einem Differenzverstärker mit ohmscher Rückkopplung ($R_2$) zur Erhöhung des Messbereichs dazwischengeschaltet ist, wobei der negative Eingang der Verstärkerkette an dem Längswiderstand ($R_1$) und der positive Eingang wie der der Verstärkerstufe am Bezugspotential (0) liegt.

**Claims**

**1.** Electronic apparatus for detecting conductance, comprising:

2*2n components ($R_m$, $R_{Re}$), which are electrically connected to form a ring and which each generally have a variable electrical conductance, n being any desirable natural number,

2k changeover switches ($U_k$) which, at the input, connect-through to their output, depending on the switch position of one of two potentials, a reference voltage ($V_{ref}$) and a reference potential (0), k being a natural number,

21 electronic channels ($V_1$) for the current-to-voltage transformation of the current, flowing through a connected component ($R_m$ or $R_{Re}$) which has just been supplied with current, each channel ($V_1$) having a negative input and a positive input as well as two outputs, 1 being a natural number, and

an A/D converter ($\mu C$), at the input bar of which the two respective outputs of the 21 channels are placed,

wherein

$$2k * 2l = 2 * 2n \text{ applies,}$$

the output of a first changeover switch ($U_1$) lies at the common point of two components ($R_{m1}$, $R_{m2}$) adjacent in the ring, a first pair of components ($R_{P1}$), the output of the next changeover switch ($U_2$) lies at the common point of the next two components ($R_{m3}$, $R_{m4}$) of the following second pair of components ($R_{P2}$), in the ring, etc., until all of the common points of the 2n pairs of components ($R_{Pn}$) of the ring are successively connected, and in consequence each respective output of the 2k changeover switches ($U_k$) is occupied n/k times,
each electronic channel ($V_1$) is connected with its negative input to a common point of two adjacent pairs of components ($R_{P1}$, $R_{P1+1}$), its positive input always lies at the reference potential (0), and the two respective outputs are placed upon the input bar of an A/D converter ($\mu C$),

the reference voltage ($V_{ref}$) during a measuring interval is always applied only to one output of the 2k changeover switches ($U_k$), so that the pairs of components connected thereto are solely supplied therewith, the remaining outputs at this time lie at the reference potential (0), and the respective negative input of the 21 electronic channels ($V_1$) is placed at the common point of two adjacent pairs of components ($R_{P1}$, $R_{P1+1}$), so that, even with multiple occupancy of the negative input, the current through one component ($R_m$ or $R_{Re}$) per electronic channel ($V_1$) is only ever measured, and

n/k pairs of components ($R_P$) are simultaneously supplied with current, and hence 21 components ($R_m$ and/or $R_{Re}$) are simultaneously measured.

**2.** Electronic apparatus according to claim 1,
**characterised in that**
the components ($R_m$) are semiconducting sensors, which change their conductance in a gas-specific manner in an atmosphere of different gas components, and
the components ($R_{Re}$) are ohmic resistors, reference resistors, which have a fixed ohmic resistance, generally a respective variable ohmic resistance, which is not influenced in respect of its conductance by a surrounding atmosphere.

**3.** Electronic apparatus according to claim 2,
**characterised in that**,
in each apparatus, the input of the respective channel ($V_1$) leads initially to the current-to-voltage transformation on the negative input of an amplifier stage, the direct output of which, on the one hand, feeds back to its negative input via an ohmic resistance ($R_{ref}$) and, on the other hand, forms one of the two outputs of the channel, and furthermore, via an ohmic longitudinal resistor ($R_1$) with an adequate measuring range, is inserted directly between the second output or another amplifier chain, comprising at least one differential amplifier with ohmic feedback ($R_2$), to increase the measuring range, the negative input of the amplifier chain lying at the longitudinal resistor ($R_1$), and the positive input, like that of the amplifier stage, lying at the reference potential (0)

**Revendications**

**1.** Dispositif électronique pour mesurer la conductance, consistant en :

- 2*2n composants ($R_m$, $R_{Re}$) connectés électriquement à un anneau, n étant un entier naturel quelconque, et qui ont en général respectivement une conductance électrique variable,
- 2k commutateurs inverseurs ($U_k$), k étant un entier naturel, qui selon la position du commutateur de l'un de deux potentiels, d'une tension de référence ($V_{ref}$) et d'un potentiel de référence (0), se connectent au début à sa sortie,
- 21, canaux électroniques ($V_1$), *l* étant un entier naturel, pour la transformation de la tension électrique du courant circulant à travers un composant raccordé et un composant tout juste alimenté en courant ($R_m$, ou $R_{Rc}$), chaque canal ($V_1$) ayant une entrée négative et une entrée positive ainsi que deux sorties,
- un convertisseur A/N ($\mu$C), dont les barrettes d'entrée comportent chaque fois les deux sorties des 2L canaux, avec l'équation, suivante :

$$2k*2l = 2*2n,$$

et dans lequel la sortie d'un premier commutateur inverseur ($U_1$) se trouve au point commun de deux composants ($R_{m1}$, $R_{m2}$) voisins dans l'anneau d'une première paire de composants ($R_{P1}$), la sortie du commutateur inverseur suivant ($U_1$) se trouve au point commun des deux composants suivants ($R_{m3}$, $R_{m4}$), de la deuxième paire suivante de composants ($R_{P2}$) dans l'anneau, etc., jusqu'à ce que tous les points communs des 2n paires de composants ($R_{Pn}$) de l'anneau soient successivement raccordés et afin que la sortie respective des 2k commutateurs inverseurs ($U_k$) n/k-fois soit occupée, chaque canal électronique ($V_1$) est raccordé par son entrée négative à un point commun de deux paires voisines de composants ($R_{P1}$, $R_{P1+1}$), son entrée positive se trouve constamment sur le potentiel de référence (0), et les deux sorties sont respectivement placées sur la barrette d'entrée d'un convertisseur A/N ($\mu$C), la tension de référence ($V_{ref}$) est placée pendant un intervalle de mesure constamment sur une sortie des 2k commutateurs inverseurs ($U_k$), de sorte que les paires de composants qui y sont raccordées soient alimentées en courant uniquement par ceux-ci, le reste des sorties se trouve à cet instant sur le potentiel de référence (O), et l'entrée négative respective des 2*l* canaux électro-

niques ($V_1$) est placée sur le point commun de deux paires voisines de composants ($R_{P1}$, $R_{P1+1}$), de sorte que même en cas d'occupation à plusieurs reprises de l'entrée négative, seul le courant circulant à travers un composant ($R_m$ ou $R_{Re}$) est mesuré par le canal électronique ($V_1$), n/k paires de composants ($R_p$) sont simultanément alimentées en courant et en conséquence, 21 composants ($R_m$ et/ou $R_{Re}$) sont mesurés simultanément.

2. Dispositif électronique selon la revendication 1,
   **caractérisé en ce que**
   les composants ($R_m$) sont des capteurs semi-conducteurs, qui modifient leur conductance, par la spécificité des gaz, dans une atmosphère comportant différents composantes gazeuses, et
   les composants ($R_{Rc}$) sont des résistances ohmiques, des résistances de référence, qui ont une résistance ohmique fixe, en général chaque fois différente, laquelle n'est pas influencée dans sa conductance par une atmosphère environnante.

3. Dispositif électronique selon la revendication 1,
   **caractérisé en ce que**
   dans chaque dispositif, l'entrée du canal respectif ($V_1$) mène d'abord à l'entrée négative d'un étage d'amplification du convertisseur de tension électrique, dont la sortie directe, d'une part, effectue un rétro-couplage à son entrée négative par l'intermédiaire d'une résistance ohmique ($R_{ref}$) et forme, d'autre part, une des deux sorties du canal, et en outre, directement la seconde sortie par l'intermédiaire d'une résistance longitudinale ohmique ($R_1$) en cas de zone de mesure suffisante, ou alors une chaîne d'amplificateur supplémentaire d'au moins un amplificateur différentiateur avec rétro-couplage ohmique ($R_2$) est intercalée pour augmenter la zone de mesure, l'entrée négative de la chaîne d'amplificateur étant située sur la résistance longitudinale ($R_1$) et l'entrée positive comme celle de l'étage d'amplification étant située sur le potentiel de référence (O).

Fig. 1

Fig. 2

# Fig. 3